# EUROPEAN PATENT SPECIFICATION

(11) **EP 1 232 746 B1**
(45) Date of publication and mention of the grant of the patent: **14.06.2006**
(21) Application number: 01200507.0
(22) Date of filing: 14.02.2001
(51) Int. Cl.: A61K 9/10, A61K 47/36

(54) **Pharmaceutical composition comprising xanthan gum**
Pharmazeutische Zusammensetzung enthaltend Xanthangummi
Composition pharmaceutique contenant de la gomme de xanthane

(43) Date of publication of application: 21.08.2002
(73) Proprietor: Forte IQ B.V., 6584 AC Molenhoek (NL)
(72) Inventor: Meijlink, Peter, 6584 AL Molenhoek (LB) (NL); Van de Lockand, Petrus Richardus Marinus, 5434 SW Vianen (NL)
(74) Representative: De Hoop, Eric

(56) References cited:
- EP-A- 0 298 740
- EP-A- 0 352 190
- EP-A- 0 405 930
- WO-A-88/10110
- US-A- 5 688 529
- US-A- 5 712 310
- US-A- 5 759 579
- US-A- 6 074 987
- US-A- 6 087 306

## Description

The present invention relates to a readily suspendible dry powder mixture composition comprising a pharmacologically active substance. The invention further relates to the use of a dry powder mixture composition for the preparation of a suspension of a pharmacologically active substance. The invention also relates to a liquid or semi-liquid pharmaceutical composition comprising a suspension of a pharmacologically active substance in an aqueous vehicle.

For many pharmacologically active substances oral administration is a preferred route of administration. In case of oral administration of pharmacologically active substances it is therapeutically and medically preferred and in some case required that the active substance after administration disintegrates to a dispersed form in the previously determined area of the gastrointestinal tract, to enhance biological availability of the active substance.

In case of oral administration of insoluble or poorly soluble or poorly wettable active substances the above described dispersed form is obtained when suspensions of the active substance in a vehicle, normally an aqueous vehicle, in particular water, are prepared.

Suspension formulations are very often presented in a multi-dose form. The vast majority of pharmaceuticals in multi-dose form have to be measured dosed by the patient from a large bottle. Suspension formulations of active substances frequently require administration two or three times per 24 hours meaning that in case of suspension formulations in multi-dose form the patient has to measure out individual doses from a large quantity of liquid.

Suspended solids tend to deposit and therefore multi-dose suspension formulations always require shaking before use to ensure homogeneity of the measured dose. Sometimes the deposited active substance even forms a cake which cannot be resuspended. Further, suspension formulations frequently require refrigeration, are inconvenient to carry when travelling and entail the possibility of inaccurate measurement and dosing and also entail the risk of undesired therapy interruption.

Suspension formulations have a limited stability and the vast majority of suspension formulations require the addition of common pharmacologically accepted preservatives. However large groups of patient have developed hypersensitivity for such preservatives, such as e.g. hydroxybenzoate esters (parabenes).

Even when preservatives are present the shelf life of liquid formulations is limited, typically 1 to 3 months, and after opening, on refrigeration, up to 10 days.

Dry formulations comprising a pharmacologically active substance optionally mixed with pharmaceutically acceptable excipients for suspension in water immediately before use are used. In case of many insoluble or poorly wettable substances suspending a dry composition comprising the active substance proceeds very difficult and slow.

WO-A-88/10110 discloses a dental powder comprising xanthan gum (Rhodigel® 200) the fillers xylitol and sorbitol sodium lauryl sulfate as surfactant and tricalcium phosphate as active ingredient.

It is therefore an object of the present invention to provide a readily suspendible dry powder mixture composition comprising a pharmacologically active substance which can be easily, very rapidly and completely suspended in an aqueous medium to form a stable suspension.

It is a further object of the invention to provide a readily suspendible dry powder mixture composition comprising a pharmacologically active substance allowing to prepare stable suspensions of pharmacologically active substances that until now could not be brought in suspension.

It is a still further object of the invention to provide a readily suspendible dry powder mixture composition comprising a pharmacologically active substance enabling to prepare suspensions with viscosities varying over a wide range.

It is yet another object of the invention to avoid the use of preservatives in preparing suspension formulations to prevent the risk of allergic reactions.

According to the present invention readily suspendible dry powder mixture compositions of a large number of insoluble or poorly wettable active substances can be prepared by mixing the active substance with a) a xanthan gum, b) a filler, preferably a sugar or sugar alcohol and c) a wetting agent or surfactant.

In one aspect the invention provides a readily suspendible dry powder mixture composition comprising
a) one or more gellants or thickeners, comprising at least one xanthan gum having such a particle size distribution that 100% by weight of the particles passes a 60 mesh sieve, 95% by weight of the particles passes a 80 mesh sieve and 70% by weight of the particles passes a 200 mesh sieve,
b) one or more fillers,
c) one or more wetting agents and/or surfactants.
d) one or more pharmacologically active substances
e) optionally one or more further pharmaceutically acceptable excipients.

In a further aspect the invention provides the use of the above described composition for the preparation of a suspension of the pharmacologically active substance in an aqueous vehicle/medium.

In a further aspect the invention provides a liquid or semi-liquid pharmaceutical composition comprising a suspension of
a) one or more gellants or thickeners, comprising at least one xanthan gum having such a particle size distribution that 100% by weight of the particles passes a 60 mesh sieve, 95% by weight of the particles passes a 80 mesh sieve and 70% by weight of the particles passes a 200 mesh sieve,
b) one or more fillers,
c) one or more wetting agents and/or surfactants,
d) one or more pharmacologically active compounds, and
e) optionally one or more further pharmaceutically acceptable excipients,
in an aqueous vehicle/medium.

In a further aspect the invention provides a dry powder mixture comprising
a) one or more gellants or thickeners, at least partially consisting of a xanthan gum having such a particle size distribution that 100% by weight of the particles passes a 60 mesh sieve, 95% by weight of the particles passes a 80 mesh sieve and 70% by weight of the particles passes a 200 mesh sieve,
b) one or more fillers, and
c) one or more wetting agents and/or surfactants.

Tests have shown that by using the present dry powder mixture composition insoluble and very poorly wettable pharmacologically active substances can be suspended very easily and in high concentrations in aqueous media. For example, by using the present invention highly concentrated suspensions (4 g mixture containing about 2 g active substance per 10 ml suspension) of Pivampicilline base, an insoluble and very poorly wettable active substance, which until now was not available as an oral suspension in generic dosage form, can be prepared for oral administration to horses. A highly concentrated Pivampicilline base dosage was only available as an injection formulation for veterinary use. Clinical acceptance studies have shown that the oral route is less painful and more accepted by horses.

Tests have also shown that by using the present invention insoluble active substances having a relatively high density such as e.g. conventional aluminium-magnesium salts, in particular aluminium oxide and magnesium hydroxide dried gels, co-precipitates and mixtures thereof, are readily suspendible in high concentrations in cold water.

The particles of the active substance to be suspended may be rather large, e.g. up to a millimetre or even more. The particles of the active substance to be suspended may be coated to obtain controlled release of the active substance.

The dry powder mixture composition of the present invention is also suitable for preparing oral formulations of poorly soluble active substances that are chemically and/or physically unstable in aqueous suspension, such as e.g. amino acids, vitamins and trace elements.
The dry powder mixture composition of the invention is also suitable for preparing more convenient uni-doses formulations of soluble active substances which are currently available in solution, such as carbocysteine and acetylcysteine, and emollients and mucus dissolving active substances e.g. herbs, phytopharmaceuticals, and homeopathics, which are easily suspendible or dispersible in aqueous media. This creates the possibility to combine all desired mixtures of insoluble, poorly soluble, poorly wettable, and soluble active substances in one dosage form, and to prolong the shelf life thereof compared with currently available products, at the same time minimising the use of preservatives. The invention also make it possible to combine active substances which in combination are chemically or physically instable in pre-prepared ready aqueous solutions or suspensions, such as the combination of organic salts with vitamins or aminoacids, for example iron salts with vitamins.

The dry powder mixture composition of the invention can be prepared by using standard pharmaceutical techniques for preparing dry mixtures. For example, the present dry powder mixture composition may be prepared by simple dry sieving and mixing the components, e.g. in a conventional sieving and tumbling mixing system. The present dry powder mixtures compositions do not require expensive specific manufacturing equipment and handling for their preparation.

The dry powder mixture compositions of the invention may be prepared by mixing all the constituent components, i.e. the pharmacologically active substance, the gellant/thickener, the filler and the wetting agent/surfactant together, wherein the components may be added in any order. Preferably the components are added in the order of concentration, e.g. triturated with the filler.

Alternatively, first a mixture of the gellant, the filler and the wetting agent may be prepared, which mixture subsequently is mixed with the pharmacologically active substance.

The present compositions are stored in a dry form. Consequently they require no or considerable less preservatives compared with liquid formulations containing the active substance, which is advantageously from a medical point of view and also results in a reduction of the costs of the administration of the active substance.

The gellant/thickener of the dry powder mixture composition of the invention comprises at least one xanthan gum. Xanthan gums have a cellulosic backbone of which each alternate glucose residue is substituted by trisaccharide side chains. Xanthan gums are ionic polymers which are soluble in both and hot cold water, but insoluble in most organic solvents. Xanthan gums in general have molecular weights more than 1 10⁶ and have a rather narrow molecular weight distribution compared with most other polysaccharides. Xanthan gums usually are in the form of the potassium, sodium or calcium salt.

To obtain the effect of the invention the xanthan gum should have such a particle size distribution as measured by means of the standard Tyler Screen Method that 100% by weight of the particles passes a 60 mesh sieve, 95% by weight of the particles passes a 80 mesh sieve and 70% by weight of the particles passes a 200 mesh sieve.

Upon dispersing the gum and surfactant in water very rapidly a highly viscous suspension should be formed. Using the following method rapidly, introducing 0.3% by weight gum (containing 0.6% surfactant) predispersed on dry sugar (2%) in water followed by 20 seconds stirring at 500 rpm and measuring the viscosity by means of a Brookfield DV-1, S1 + 260 rpm, the viscosity after 2.5 minutes should be in the range of 100 to 205 mPa.s, preferably in the range of 125 to 200 mPa.s and most preferably in the range of 150 to 175 mPa.s.

Examples of suitable xanthan gums are the products marketed on the trade names Ferwogel® type 30.385 or Rhodigel Ultra® . Ferwogel® type 30.385 has a molecular weight of 3.5 to 4.0 10⁶.

In general it is preferred to use as thickener/gellant in the dry powder mixtures of the invention only the above defined xanthan gum. However, the dry powder mixture composition of the invention may comprise further gellants/thickeners in addition to the xanthan gum, in particular if a specific therapeutical effect is aimed at. As additional gellants/thickeners any pharmaceutically acceptable gellant/thickener may be used. Non-limiting examples of additional gellants/thickeners are polysaccharide gums, hydrocolloids, proteins and starches such as gelatine, caseinate, locust bean gum, whey protein, gellan carageenan, agar, alginate, HM pectin, LMC pectin, LMA pectin, CMC, MCC, guar gum, LBG taragum, konjac, gum arabic, soy protein, carboxyvinylpolymers, polyethylene glycol carbomer, magnesium-aluminium salts (for example Veegum) colloidal silicon oxides and mixtures thereof.

Preferably xanthan gum is present in the dry powder mixture composition of the invention in a content of 0.01 to 80% by weight of the mixture.

The filler of the dry powder mixture composition of the invention may be any pharmacologically acceptable filler. Preferably the filler is a sugar or sugar alcohol. Most preferred are sugars and sugar alcohols having free-flowing properties and a particle size matching or in line with the other components to ensure a homogeneous mixture. Non-limiting examples of suitable fillers are mannitol, sorbitol, sucrose, lactose cellulose derivatives, MCC, calcium phosphates, tricalcium phosphate, dicalcium phosphate and mixtures thereof.

Preferably the sugar or sugar alcohol is present in the dry powder mixture composition of the invention in a content of 10 to 75% by weight. Using a high content of sugar or sugar alcohol results in an high flowability of the final dry mixture. A high content of sugar or sugar alcohol may also contribute to a lump free wettability of dry powder mixtures containing high concentrations of xanthan gums.

As further essential component the dry powder mixture composition of the invention comprises at least one wetting agent or surfactant. The wetting agent or surfactant may be any type of wetting agent or surfactant commonly used in the pharmaceutical technique for oral and topical formulations. The surfactant may be of the ionic, nonionic or amphoteric type. Non-limiting examples of wetting agents and surfactants suitable for use in the dry powder mixture composition of the invention are polyethylene glycols, hydroxypolyether derivatives, dioctyl sodium sulfosuccinate, sodium and calcium lauryl sulphate, sorbitan fatty acid esters polyoxyethylene sorbitan monolaurate, monopalmitate, monostearate, monooleate, tristearate and mixtures thereof.

The wetting agent or surfactant is preferably a polyethylene glycol/macrogol, in particular a solid macrogol-polyethylene glycol 2000, 3000, 4000, 6000, 8000, preferably in the form of a powder.

Preferably the wetting agent or surfactant is present in the dry powder mixture composition of the invention in a content of 0.1 to 50% by weight.

Preferably the wetting agent or surfactant is mixed with the other components by applying it in a finely dispersed form one of the other components or a mixture of two or more of thereof.

Non-limiting examples of the active substances to be incorporated in the dry powder mixture are Algeldrates, Malgeldrates, calcium carbonate, magnesium and sodium alginate, aluminium hydroxide-magnesium carbonate co-precipitated (dried) gel, magnesium carbonate, potassium and sodium bicarbonates, magnesium trisilicates, and mixtures thereof including other antacids, antibiotics, analgetics, antipyretics, antiviral drugs, H+blockers, antihistaminica, laxantia. The active substance may also be a food supplement such as amino acids, vitamins, trace elements

Preferably the dry powder mixture composition of the invention comprises between 0.1 and 79.99 % by weight of active substances. The may contain one active substance or a mixture of two or more active substances.

The dry powder mixture composition of the invention may comprise further usual pharmaceutical additives such as disintegrating agents, lubricant, sweetening agents, flavouring agents etc. provided that these additives doe not affect the suspendibility of the composition of the invention.

The dry powder mixture of the invention is preferably presented as a measured dose composition for reconstitution of a suspension, intended for immediate use.The dry powder mixture composition of the invention may be filled into any suitable pharmaceutical presentation form. Non-limiting examples of presentation forms are sachets, bottles, plastic syringes etc.

The dry powder mixture compositions of the invention are used for preparing a liquid formulation usually in the form of a suspension of the active substance. The suspension formulations of the invention are prepared by admixing the dry powder mixture composition of the invention with a liquid vehicle, preferably water or glycol or a mixture thereof. Typically the suspension is prepared by suspending the composition in cold water by adding the dry powder mixture composition to a glass of (tap) water (approx. 100 ml) or by adding water to a glass containing the powder mixture, and minimal shaking or agitating. For the preparation of larger doses of the active substance a suspension is prepared by adding a measured dose of tape water to a bottle, syringe etc., pre-filled with the dry powder mixture composition of the invention, followed by minimal shaking to obtain complete suspension of the solid components.

The liquid vehicle used in preparing the suspension formulations of the invention is admixed with the dry powder mixture in an amount sufficient to obtain the desired concentration of the active substance and viscosity. Depending on the relative amounts and nature of the components of the dry mixture composition the consistency of the suspension/liquid formulation prepared there from can vary from that of a liquid having a very low viscosity to that of an very viscous, almost semi-solid gel.

The viscosity of the suspension can be adjusted easily either by changing the concentration of the components or by adding a viscosity enhancing agent such as for example sodium chloride, guar gum, or locus bean gum to the dry powder mixture.

The suspensions of the invention may be prepared in the form of a highly or medium viscous gel, suitable for the topical or rectal administration of pharmacologically active substances which are absorbed through the skin, or are intended to have a local therapeutically effect respectively, for example analgesics, anti haemorrhoids, anti flogistics, antihelmetics, anti emetics etc.

The invention will now be illustrated by way of the following examples.

The following examples are illustrative of the processes that may be used for preparing highly concentrated suspensions of water insoluble and poorly wettable active substances.

### EXAMPLE 1

Dry powder mixture composition for veterinary suspension containing a measured high dose of Pivampicilline base (4 gram mixture per 10 ml)

### Preparation of dry powder mixture

To 1 kg of Pivampicilline base, 1 kg of Polyethylene glycol 600 powder and 20 g of colloidal silicon oxide, 75 g of Ferwogel® type 30.385 containing about 0.6 % polysorbate, 5 g of sodium chloride and 5 g flavouring agents were added, the obtained mixture was passed through a Frewitt 1.25 mm sieve to form a lump free powder mixture. The sieved mixture was placed in an Erweka tumble mixture and thoroughly blended for 10 minutes.

### Application for juvenile or pony horses (up to 300 kg body weight; dosage 20 mg/kg horse)

12.613 g of the above prepared sieved and blended mixture was filled into a plastic syringe (volume of 100 ml) and 30 ml of tap water was added. The syringe was shaken during 3 minutes and left standing for 2 minutes. The solid components had suspended completely to form a highly viscous suspension which was ready for administration.

### Application for adult or regular sized horses (up to 600 kg body weight; dosage 20 mq/kg horse)

25.226 g of the above prepared sieved and blended mixture was filled into a plastic syringe (volume of 140 ml) and 60 ml of tap water was added. The syringe was shaken during 3 minutes and left standing for 2 minutes. The solid components had suspended completely to form a highly viscous suspension which was ready for administration.

### EXAMPLE 2

Dry powder compositions for suspensions containing a regular measured dose of an antacid, magnesium oxide and aluminium hydroxide co-precipitated powder for administration to humans.

### Preparation of the powder mixture

To 2.254 kg of co-precipitated magnesium oxide and aluminium hydroxide powder comprising 28% by weight polyols, 300 g Ferwogel® type 30.385 containing about 0.6 % polysorbate, and 25 g of flavouring agents were added. The obtained mixture was passed through a Frewitt 1.25 mm sieve to form a lump free powder mixture and the powder mixture was placed in an Erweka mixer and thoroughly blended for 10 minutes.

### Administration of measured dosages in sachets

1.302 g of the resulting blend was filled into a sachets. A drinking glass was filled with tap water (about 100 ml) and the content of the sachet was added. After mild agitation during about 30 seconds a complete suspension ready for administration was obtained.

## Claims

1. A dry powder mixture composition comprising
a) one or more gellants or thickeners, comprising at least one xanthan gum having such a particle size distribution that 100% by weight of the particles passes a 60 mesh sieve, 95% by weight of the particles passes a 80 mesh sieve and 70% by weight of the particles passes a 200 mesh sieve,
b) one or more fillers,
c) one or more wetting agents and/or surfactants.
e) optionally one or more further pharmaceutically acceptable excipients.

2. A dry powder mixture composition according to claim 1 wherein the xanthan gum is a gum which upon rapidly introducing 0.3% by weight thereof predispersed on dry sugar (2%) in water followed by 20 seconds stirring at 500 rpm and measuring the viscosity by means of a Brookfield DV-1, S1 + 260 rpm, provides a suspension viscosity after 2.5 minutes in the range of 100 to 205 mPa.s, preferably in the range of 125 to 200 mPa. and most preferably in the range of 150 to 175 mPa.s.

3. A dry powder mixture composition according to claim 1 or claim 2 comprising
a) 0.01 to 80 % by weight of the one or more gellants or thickeners.
b) 10 to 75% by weight of the one or more fillers,
and
c) 0.1 to 50% by weight of one or more wetting agents and/or surfactants.

4. A dry powder mixture composition according to claim 1 or claim 2 further comprising
d) one or more pharmacologically active substances.

5. A dry powder mixture composition according to claim 3 further comprising
d) 0.01 to 79,99 % by weight of one or more active substances.

6. A dry powder mixture composition according to claim 4 or claim 5 comprising 0.1 to 60% by weight of the active substance.

7. The use of a composition according to any one of the claims 1 to 6 for the preparation of a suspension of a pharmacologically active substance in an aqueous vehicle/medium.

8. A pharmaceutical composition prepared by suspending a composition according to any one of the claims 1 to 6 in an aqueous vehicle.

9. A liquid or semi-liquid pharmaceutical composition comprising a suspension of
a) one or more gellants or thickeners, comprising at least one xanthan gum having such a particle size distribution that 100% by weight of the particles passes a 60 mesh sieve, 95% by weight of the particles passes a 80 mesh sieve and 70% by weight of the particles passes a 200 mesh sieve,
b) one or more fillers,
c) one or more wetting agents and/or surfactants,
d) one or more pharmacologically active compounds, and
e) optionally one or more further pharmaceutically acceptable excipients,
in an aqueous vehicle/medium.

10. A liquid or semi-liquid pharmaceutical composition according to claim 9, comprising, based on the components a), b), c), and d), and optionally e)
a) 0.01 to 80 % by weight of the one or more gellants or thickeners,
b) 10 to 75 % by weight of the one or more fillers,
c) 0.1 to 50 % by weight of the one or more wetting agents and/or surfactants,
d) 0.1 to 79,99 % by weight of the one or more pharmacologically active compounds, in an aqueous vehicle/medium.

## Patentansprüche

1. Trockene Pulvermischungzusammensetzung umfassend:
a) ein oder mehrere Geliermittel oder Verdickungsmittel, umfassend zumindest ein Xanthangummi mit einer derartigen Kongrößenverteilung, daß 100 Gewichtsprozent der Körner ein Sieb von 60 mesh passiert, 95 Gewichtsprozent der Körner ein Sieb von 80 mesh passiert und 70 Gewichtsprozent der Körner ein Sieb von 200 mesh passiert,
b) einen oder mehrere Füllstoffe,
c) ein oder mehrere Benetzungsmittel und/oder Tenside,
d) gegebenenfalls eine oder mehrere andere pharmazeutisch aktzeptabele Träger.

2. Trockene Pulvermischungzusammensetzung nach Anspruch 1, wobei das Xanthangummi ein Gummi ist, das bei schnell Einführen von 0,3 Gewichtsprozent davon, auf trockenem Zucker (2%) in Wasser dispergiert, durch 20 Sekunden Rühren bei 500 rpm gefolgt, und das Messen der Viskosität durch ein Brookfield DV-1, S1 + 260 rpm, eine Suspensionsviskosität nach 2,5 Minuten im Bereich von 100 bis 205 mPa.s ergibt, vorzugsweise im Bereich von 125 bis 200 mPa. und am liebsten im Bereich von 150 bis 175 mPa.s.

3. Trockene Pulvermischungzusammensetzung nach Anspruch 1 oder 2, umfassend:
a) 0,01 bis 80 Gewichtsprozent von dem einen oder mehreren Geliermitteln oder Verdickungsmitteln,
b) 10 bis 75 Gewichtsprozent von dem einen oder mehreren Füllstoffen,
c) 0,1 bis 50 Gewichtsprozent von dem einen oder mehreren Benetzungsmitteln oder Tensiden.

4. Trockene Pulvermischungzusammensetzung nach Anspruch 1 oder Anspruch 2 weiter umfassend
d) eine oder mehrere pharmazeutisch aktive Substanze.

5. Trockene Pulvermischungzusammensetzung nach Anspruch 3 weiter umfassend
d) 0,01 bis 79,99% Gewichtsprozent von einer oder mehreren aktiven Substanzen.

6. Trockene Pulvermischungzusammensetzung nach Anspruch 4 oder Anspruch 5, umfassend 0,1 bis 60% Gewichtsprozent der aktiven Substanz.

7. Gebrauch einer Zusammensetzung nach einem oder mehreren der Ansprüche 1 bis 6 für die Bereitung einer Suspension einer pharmazeutisch aktiven Substanz in einem wässerigen Vehikel/Medium.

8. Pharmazeutische Zusammensetzung durch das Suspendieren einer Zusammensetzung nach einem der Ansprüche 1 bis 6 in einem wässerigen Vehikel bereitet.

9. Flüssige oder semi-flüssige pharmazeutische Zusammensetzung, umfassend eine Suspension von
a) ein oder mehrere Geliermittel oder Verdickungsmittel, umfassend zumindest ein Xanthangummi mit einer derartigen Kongrößenverteilung, daß 100 Gewichtsprozent der Körner ein Sieb von 60 mesh passiert, 95 Gewichtsprozent der Körner ein Sieb von 80 mesh passiert und 70 Gewichtsprozent der Körner ein Sieb von 200 mesh passiert,
b) einen oder mehrere Füllstoffe,
c) ein oder mehrere Benetzungsmittel und/oder Tenside,
d) eine oder mehrere pharmazeutisch aktive Verbindungen, und
e) gegebenenfalls eine oder mehrere pharmazeutisch akzeptabele weitere Träger,
in einem wässerigen Vehikel/Medium.

10. Flüssige oder semi-flüssige pharmazeutische Zusammensetzung nach Anspruch 9, umfassend, auf den Komponenten a), b), c), d) und gegebenenfalls e) basiert
a) 0,01 bis 80 Gewichtsprozent von dem einen oder mehreren Geliermitteln oder Verdickungsmitteln,
b) 10 bis 75 Gewichtsprozent von dem einen oder mehreren Füllstoffen, c) 0,1 bis 50 Gewichtsprozent von dem einen oder mehreren Benetzungsmitteln oder Tensiden,
d) 0,1 bis 79,99 Gewichtsprozent von der einen oder mehreren pharmazeutisch aktiven Verbindungen, in einem wässerigen Vehikel/Mittel.

## Revendications

1. Composition de mélange de poudres sèches, comprenant
a) un ou plusieurs gélifiants ou épaississants comprenant au moins une gomme xanthane ayant une distribution de tailles de particules telle que 100 % en poids des particules passent à travers un tamis de 60 mesh, 95 % en poids des particules passent à travers un tamis de 80 mesh et 70 % en poids des particules passent à travers un tamis de 200 mesh,
b) une ou plusieurs charges,
c) un ou plusieurs agents mouillants et/ou tensioactifs,
e) facultativement, un ou plusieurs autres excipients pharmaceutiquement acceptables.

2. Composition de mélange de poudres sèches selon la revendication 1, dans laquelle la gomme xanthane est une gomme qui, après l'introduction rapide dans l'eau, de 0,3 % en poids de celle-ci, préalablement dispersée sur du sucre sec (2 %), suivie de 20 secondes d'agitation à 500 tours/minute et mesure de la viscosité au moyen d'un appareil Brookfield DV-1, S1 + 260 tours/minute, fournit une viscosité de suspension après 2,5 minutes dans la plage de 100 à 205 mPa.s, de préférence dans la plage de 125 à 200 mPa, et de façon plus préférée dans la plage de 150 à 175 mPa.s.

3. Composition de mélange de poudres sèches selon la revendication 1 ou la revendication 2, comprenant
a) 0,01 à 80 % en poids d'un ou plusieurs gélifiants ou épaississants,
b) 10 à 75 % en poids d'une ou plusieurs charges, et
c) 0,1 à 50 % en poids d'un ou plusieurs agents mouillants et/ou tensioactifs.

4. Composition de mélange de poudres sèches selon la revendication 1 ou la revendication 2, comprenant de plus
d) une ou plusieurs substances actives pharmacologiquement.

5. Composition de mélange de poudres sèches selon la revendication 3, comprenant de plus
d) 0,01 à 79,99 % en poids d'une ou plusieurs substances actives.

6. Composition de mélange de poudres sèches selon la revendication 4 ou la revendication 5, comprenant de 0,1 à 60 % en poids de la substance active.

7. Utilisation d'une composition selon l'une quelconque des revendications 1 à 6 pour la préparation d'une suspension d'une substance active pharmacologiquement dans un véhicule/milieu aqueux.

8. Composition pharmaceutique préparée par la mise en suspension d'une composition selon l'une quelconque des revendications 1 à 6 dans un véhicule aqueux.

9. Composition pharmaceutique liquide ou semi-liquide comprenant une suspension de
a) un ou plusieurs gélifiants ou épaississants, comprenant au moins une gomme xanthane ayant une distribution de tailles de particules telle que 100 % en poids des particules passent à travers un tamis de 60 mesh, 95 % en poids des particules passent à travers un tamis de 80 mesh et 70 % en poids des particules passent à travers un tamis de 200 mesh,
b) une ou plusieurs charges,
c) un ou plusieurs agents mouillants et/ou tensioactifs,
d) un ou plusieurs composés pharmacologiquement actifs, et
e) facultativement, un ou plusieurs autres excipients pharmaceutiquement acceptables, dans un véhicule/milieu aqueux.

10. Composition pharmaceutique liquide ou semi-liquide selon la revendication 9, comprenant, rapportée aux composants a), b), c) et d) et facultativement e)
a) 0,01 à 80 % en poids d'un ou plusieurs gélifiants ou épaississants,
b) 10 à 75 % en poids d'une ou plusieurs charges,
c) 0,1 à 50 % en poids d'un ou plusieurs agents mouillants et/ou tensioactifs,
d) 0,1 à 79,99 % en poids d'un ou plusieurs composés pharmacologiquement actifs, dans un véhicule/milieu aqueux.
